# DEMANDE DE BREVET EUROPEEN

(11) **EP 1 055 707 A1**
(43) Date de publication de la demande: **29.11.2000**
(21) Numéro de dépôt: 00401421.3
(22) Date de dépôt: 23.05.2000
(51) Int. Cl.: C08L 57/12, A61K 7/06

(54) **Latex inverse auto inversible**

(30) Priorité: 28.05.1999 FR 9906768
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Carrausse, Maryse, 81100 Castres (FR); Tabacchi, Guy, 81100 Castres (FR); Mallo, Paul, 78400 Chatou (FR); Boiteux, Jean-Pierre, 81710 Saix (FR); Milius, Alain, 06000 Nice (FR); Michel, Nelly, 94700 Maisons Alfort (FR); Amalric, Chantal, 81700 Blan (FR)
(74) Mandataire: Conan, Philippe Claude

(57) **Abrégé**

Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 40% en poids, d'un polyélectrolyte linéaire, branché ou réticulé, caractérisé en ce que ledit polyélectrolyte est, soit un homopolymère à base d'un monomère possédant une fonction acide fort partiellement ou totalement salifiée, soit un homopolymère à base d'un monomère possédant une fonction acide faible partiellement ou totalement salifiée, soit un homopolymère à base d'un monomère cationique, soit un copolymère à base d'au moins un monomère possédant une fonction acide fort partiellement ou totalement salifiée, copolymérisé, ou bien avec au moins un monomère possédant une fonction acide faible partiellement ou totalement salifiée, ou bien avec au moins un monomère neutre, soit un copolymère à base d'un monomère cationique, copolymérisé, avec au moins un monomère neutre, et caractérisé en ce que l'un au moins des agents émulsifiants de type huile dans eau est un composé de formule (I) :

R₁-O-[CH(R₂)-CH₂-O]ₙ-(G)ₓ-H (I)

dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal soit à zéro soit à un nombre entier compris entre 1 et 30.

Utilisation en cosmétique.

## Description

La présente demande concerne des latex inverses auto-inversibles, leur procédé de préparation et leurs applications. L'un des avantages des latex inverses auto-inversibles, est que, se présentant sous forme liquide, ils permettent la manipulation aisée de polymères de très haute masse moléculaire. La mise en solution de ces latex est extrêmement rapide ; les polymères contenus dans ces latex inverses sont par exemple, des copolymères acrylamide/acrylate de métal alcalin, tels que ceux décrits dans la demande internationale de brevet publiée sous le numéro WO 95/35089, ou acrylamide/acrylamido 2-méthyl 2-propanesulfonate de sodium, tels que ceux décrits dans la demande de brevet européen publiée sous le numéro EP 0503853. ils sont déjà neutralisés et, lorsqu'ils sont mis en solution dans l'eau, par exemple à une concentration de 1%, on observe que le pH est généralement supérieur à 6. Cependant, l'ensemble des polymères cités a tendance à perdre leurs propriétés rhéologiques, lorsque le milieu à traiter contient des sels ; cette tendance est accentuée avec l'augmentation de la concentration en sels présents dans ledit milieu. La demanderesse a donc cherché à mettre au point des latex inverses auto-inversibles, présentant des viscosités importantes qui soient stables en présence d'électrolytes dans la phase aqueuse.

L'invention a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 40% en poids, d'un polyélectrolyte linéaire, branché ou réticulé, caractérisé en ce que ledit polyélectrolyte est, soit un homopolymère à base d'un monomère possédant une fonction acide fort partiellement ou totalement salifiée, soit un homopolymère à base d'un monomère possédant une fonction acide faible partiellement ou totalement salifiée, soit un homopolymère à base d'un monomère cationique, soit un copolymère à base d'au moins un monomère possédant une fonction acide fort partiellement ou totalement salifiée, copolymérisé, ou bien avec au moins un monomère possédant une fonction acide faible partiellement ou totalement salifiée, ou bien avec au moins un monomère neutre, soit un copolymère à base d'un monomère cationique, copolymérisé, avec au moins un monomère neutre, et caractérisé en ce que l'un au moins des agents émulsifiants de type huile dans eau est un composé de formule (I) :

R₁-O-[CH(R₂)-CH₂-O]ₙ-(G)ₓ-H (I)

dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30

Par "agent émulsifiant du type eau dans huile", on désigne des agents émulsifiants possédant une valeur HLB suffisamment faible pour fournir des émulsions eau dans huile, tels que les polymères tensioactifs commercialisés sous le nom de HYPERMER™ ou tels que les esters de sorbitan, comme le monooléate de sorbitan commercialisé par la Société SEPPIC sous le nom Montane™ 80 ou l'isostéarate de sorbitan commercialisé par SEPPIC sous le nom Montane™ 70. Lorsqu'il s'agit d'un mélange d'agents émulsifiants du type eau dans huile, la valeur HLB à prendre en considération est celle dudit mélange.

Par "agent émulsifiant du type huile dans eau", on désigne outre le composé de formule (I) tel que définie précédemment, des agents émulsifiants possédant une valeur HLB suffisamment élevée pour fournir des émulsions huile dans l'eau tels que, par exemple, les esters de sorbitan éthoxylés comme l'oléate de sorbitan éthoxylé à 20 moles d'oxyde d'éthylène, ou le laurate de sorbitan éthoxylé à 20 moles d'oxyde d'éthylène commercialisés par la société SEPPIC respectivement sous les noms MONTANOX™ 80 B, et MONTANOX™ 20, l'alcool laurique éthoxylé à 7 moles d'oxyde d'éthylène , l'huile de ricin éthoxylée à 40 moles d'oxyde d'éthylène, ou l'huile de ricin éthoxylée à 40 moles d'oxyde d'éthylène, commercialisés par la société SEPPIC sous le nom SIMULSOL™ P7 et SIMULSOL™ OL 50 ou les nonylphénols éthoxylés. Lorsqu'il s'agit d'un mélange d'agents émulsifiants du type huile dans eau, la valeur HLB à prendre en considération est celle dudit mélange. Selon un aspect particulier de la présente invention, lorsque la composition selon l'invention comprend un mélange d'agents émulsifiants de type huile dans eau, (H/E), au moins 50% en poids de ce mélange est constitué de composés de formule (I) telle que définie précédemment.

Par partiellement ou totalement salifiée, on signifie que les fonctions acide fort ou acide faible sont partiellement ou totalement salifiées sous forme, notamment de sel de métal alcalin, tel que le sel de sodium ou le sel de potassium, de sel d'ammonium (NH₄⁺) ou de sel d'aminoalcool tel que le sel de monoéthanolamine (HOCH₂CH₂NH₃⁺).

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La fonction acide fort du monomère en comportant, est notamment la fonction acide sulfonique ou la fonction acide phosphonique, lesdites fonctions étant partiellement ou totalement salifiées. Ledit monomère peut être par exemple, l'acide styrènesulfonique, le méthacrylate de (2-sulfo éthyle) ou l'acide styrène-phosphonique, partiellement ou totalement salifiée. Lorsque le monomère comporte une fonction acide fort, il s'agit de préférence de l'acide 2-méthyl 2-[(1-oxo 2-propènyl) amino] 1-propane-sulfonique (AMPS) partiellement ou totalement salifié sous forme de sel de sodium, de sel d'ammonium, ou de sel de monoéthanolamine.

La fonction acide faible du monomère en comportant, est notamment la fonction acide carboxylique partiellement ou totalement salifiée. Ledit monomère en comportant est notamment choisi parmi l'acide acrylique, l'acide méthacrylique, l'acide itaconique ou l'acide maléique partiellement ou totalement salifié sous forme de sel de sodium, de sel d'ammonium ou de sel de monoéthanolamine.

Lorsque le polyélectrolyte est un copolymère à base d'un monomère possédant une fonction acide fort partiellement ou totalement salifiée, copolymérisé, avec au moins un monomère neutre, ledit monomère neutre est notamment choisi parmi l'acrylamide, le méthacrylamide, le vinyl pyrrolidone, l'acrylate de (2-hydroxy éthyle), l'acrylate de (2,3-dihydroxy propyle), le méthacrylate de (2-hydroxy éthyle) ou le méthacrylate de (2,3-dihydroxy propyle) ou un dérivé éthoxylé de poids moléculaire compris entre 400 et 1000, de chacun de ces esters hydroxylés décrits ci-dessus.

Par radical hydrocarboné linéaire ou ramifié, saturé ou insaturé, comprenant de 1 à 30 atomes de carbone, on désigne pour R₁ dans la formule (I) telle que définie précédemment, notamment les radicaux alkyles ou les radicaux alkènyle. R₁ représente plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés.

Par reste d'un saccharide, on désigne pour G, un radical bivalent résultant de l'enlèvement sur une molécule de sucre, d'une part d'un atome d'hydrogène d'un groupe hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ peut se présenter sous toute forme d'isomérie, qu'il s'agisse d'isomérie optique, d'isomérie géométrique ou d'isomérie de positon, elle peut aussi représenter un mélange d'isomères.

Dans la formule (I) telle que définie précédemment, le radical R₁-O-[CH(R₂)-CH₂-O]ₙ- est lié à G, par le carbone anomérique de manière à former une fonction acétal. Le groupe divalent -[CH(R₂)-CH₂-O]ₙ- représente, soit une chaîne composée uniquement de groupes éthoxyle (R₂=H), soit une chaîne composée uniquement de groupes propoxyle (R₂=CH₃), soit une chaîne composée à la fois de groupes éthoxyle et de groupes propoxyle. Dans ce dernier cas, les fragments -CH₂-CH₂-O- et -CH(CH₃)-CH₂-O- sont distribués dans ladite chaîne, de façon séquencée ou aléatoire, x, qui représente dans la formule (I) le degré moyen de polymérisation du saccharide, est plus particulièrement compris entre 1 et 3, notamment entre 1,05 et 2,5, tout particulièrement entre 1,1 et 2,0 et de préférence ,inférieur ou égal à 1,5.

Selon un premier aspect particulier de la présente invention, G représente le reste du glucose.

Selon un deuxième aspect particulier de la présente invention, G représente le reste du xylose.

Selon un troisième aspect particulier de la présente invention, n est égal à 0.

Selon un quatrième aspect particulier de la présente invention, R₁ représente un radical comportant de 8 à 18 atomes de carbone. R₁ représente plus particulièrement un radical octyle, décyle, undécyle, dodécyle, tétradécyle ou hexadécyle, lesdits radicaux étant linéaires ou ramifiés.

Selon un cinquième aspect particulier de la présente invention, celle-ci a pour objet une composition telle que définie précédemment, dans laquelle l'agent émulsifiant de type huile dans eau est un composé de formule (I) :

R₁-O-[CH(R₂)-CH₂-O]ₙ-(G)ₓ-H (I)

ou un mélange de composés de formule (I).

Comme agent émulsifiant de type huile dans eau approprié , il y a par exemple les produits commerciaux suivants :
Le SIMULSOL™SL8, commercialisé par la société SEPPIC, qui est une solution aqueuse contenant entre environ 35% et 45% en poids d'un mélange d'alkyl polyglycosides consistant en entre 45% en poids et 55% en poids d'un composé de formule (I), dans laquelle G représente le reste du glucose, x est égal à environ 1,45, n est égal à 0 et R₁ représente un radical décyle, et entre 45% en poids et 55% en poids d'un composé de formule (I), dans laquelle G représente le reste du glucose, x est égal à environ 1,45, n est égal à 0 et R₁ représente un radical octyle ;
Le SIMULSOL™ SL10, commercialisé par la société SEPPIC, qui est une solution aqueuse contenant entre environ 40% en poids et 50% en poids d'un mélange d'alkyl polyglycosides, consistant en environ 85% en poids d'un composé de formule (I), dans laquelle G représente le reste du glucose, x est égal à environ 1,45, n est égal à 0 et R₁ représente un radical décyle, environ 7,5% en poids d'un composé de formule (I), dans laquelle G représente le reste du glucose, x est égal à environ 1,45, n est égal à 0 et R₁ représente un radical dodécyle et environ 7,5% en poids d'un composé de formule (I), dans laquelle G représente le reste du glucose, x est égal à environ 1,45, n est égal à 0 et R₁ représente un radical tétradécyle ;
Le SIMULSOL™ SL11, commercialisé par la société SEPPIC, qui est une solution aqueuse contenant entre environ 40% en poids et 50% en poids d'un mélange d'alkyl polyglycosides de formule (I), dans laquelle G représente le reste du glucose, x est égal à environ 1,45, n est égal à 0 et R₁ représente un radical undécyle ; ou
Le SIMULSOL™ SL26, commercialisé par la société SEPPIC, qui est une solution aqueuse contenant entre environ 40% en poids et 55% en poids d'un mélange d'alkyl polyglycosides consistant en environ 70% en poids d'un composé de formule (I), dans laquelle G représente le reste du glucose, x est égal à environ 1,45, n est égal à 0 et R₁ représente un radical dodécyle, environ 25% en poids d'un composé de formule (I), dans laquelle G représente le reste du glucose, x est égal à environ 1,45, n est égal à 0 et R₁ représente un radical tétradécyle et environ 5% en poids d'un composé de formule (I), dans laquelle G représente le reste du glucose, x est égal à environ 1,45, n est égal à 0 et R₁ représente un radical hexadécyle.

L'invention a plus particulièrement pour objet une nouvelle composition telle que définie précédemment, caractérisée en ce que le polyélectrolyte anionique, est réticulé et/ou branché par un agent de réticulation et/ou un agent ramification choisi parmi les composés diéthyléniques ou polyéthyléniques, et tout particulièrement parmi l'acide diallyloxyacétique ou un des sels et notamment son sel de sodium, le triallylamine, le triméthylol propanetriacrylate, le diméthacrylate d'éthylèneglycol, le diacrylate de diéthylène glycol, le diallylurée ou le méthylène bis(acrylamide). L'agent de réticulation et/ou de ramification est généralement utilisé dans la proportion molaire exprimée par rapport aux monomères mis en oeuvre, de 0,005% à 1%, notamment de 0,01% à 0,2% et plus particulièrement de 0,01% à 0,1%.

Le latex selon l'invention contient généralement de 2,5% à 15% en poids, et de préférence de 4% à 9% en poids, d'agents émulsifiants, parmi lesquels de 20% à 50%, notamment de 25% à 40% du poids total des agents émulsifiants présents sont du type eau dans huile (E/H) et parmi lesquels de 80% à 50%, notamment de 75% à 60%, du poids total des agents émulsifiants, sont du type huile dans eau (H/E).

Selon un aspect particulier, la composition, telle que définie précédemment, est caractérisée en ce que la phase huile représente de 15% à 50%, de préférence de 20% à 25%, de son poids total. Cette phase huile est constituée soit par une huile minérale commerciale contenant des hydrocarbures saturés de type paraffine, isoparaffine, cycloparaffine, présentant à température ambiante, une densité entre 0.7 et 0.9 et un point d'ébullition supérieur à 180°C, telle que par exemple, l'ISOPAR™ M, l'ISOPAR™ L, l'ISOPAR™E ou l'ISOPAR™G, l'EXXOL™ D 100 S commercialisé par EXXON ou une huile blanche minérale telle que le MARCOL™ 52, le MARCOL™82, également commercialisées par EXXON, l'isohexadécane commercialisé par BAYER ou l'isododécane, soit par une huile végétale, soit par une huile de synthèse, soit par un mélange de plusieurs de ces huiles ; on peut aussi citer l'isostéarate d'isostéaryle seul ou en mélange avec d'autres huiles. L'isohexadécane, qui est identifié dans Chemical Abstracts par le numéro RN = 93685-80-4, est un mélange d'isoparaffines en C₁₂, C₁₆ et C₂₀ contenant au moins 97% d'isoparaffines en C₁₆, parmi lesquelles le constituant principal est le 2,2,4,4,6,8,8-heptaméthyl nonane (RN = 4390-04-9). Il est commercialisé en France par la société Bayer. Le MARCOL™ 52 est une huile commerciale répondant à la définition des huiles de vaseline dans le Codex français. C'est une huile blanche minérale conforme aux règles FDA 21 CFR 178.878 et CFR 178.3620(a) ; elle est inscrite à la Pharmacopée des USA, US XXIII (1995) et à la Pharmacopée européenne (1993). Selon un aspect préféré, la composition, telle que définie précédemment, est caractérisée en ce que la phase huile consiste en de l'isohexadécane ou en une huile blanche minérale.

Les latex contiennent entre 5% et 60% en poids, d'eau et plus particulièrement entre20 % et 50 % en poids d'eau. Le latex selon l'invention peut également contenir divers additifs tels que des agents complexant, des agents de transfert ou des agents limiteurs de chaîne.

Selon un autre aspect particulier de la présente invention celle-ci a pour objet une composition telle que définie précédemment dans laquelle le polyélectrolyte anionique est soit un homopolymère de l'acide 2-méthyl 2-[(1-oxo 2-propènyl) amino] 1-propane-sulfonique (AMPS) partiellement ou totalement salifié sous forme de sel de sodium, de sel d'ammonium ou de sel de monoéthanolamine, soit un homopolymère de l'acide acrylique partiellement ou totalement salifié sous forme de sel de sodium, de sel d'ammonium ou de sel de monoéthanolamine, soit un copolymère comportant en proportion molaire de 30% à 50% d'acide 2-méthyl 2-[(1-oxo 2-propènyl) amino] 1-propanesulfonique (AMPS) partiellement ou totalement salifié sous forme de sel de sodium, de sel d'ammonium ou de sel de monoéthanolamine et de 50% à 70% d'acrylamide, soit un copolymère comportant en proportion molaire de 60% à 90% d'acide 2-méthyl 2-[(1-oxo 2-propènyl) amino] 1-propanesulfonique (AMPS) partiellement ou totalement salifié sous forme de sel de sodium, de sel d'ammonium, ou de sel de monoéthanolamine et de 10% à 40% d'acrylate de (2-hydroxy éthyle) ou soit un copolymère comportant en proportion molaire de 30% à 90% et plus particulièrement de 30 à 45% d'acide 2-méthyl 2-[(1-oxo 2-propènyl) amino] 1-propanesulfonique (AMPS) partiellement ou totalement salifié sous forme de sel de sodium, de sel d'ammonium ou de sel de monoéthanolamine et d'acide acrylique partiellement ou totalement salifié sous forme de sel de sodium, de sel d'ammonium ou de sel de monoéthanolamine.

Selon un autre aspect de la présente invention, celle-ci a aussi pour objet un procédé de préparation de la composition telle que définie précédemment, caractérisé en ce que :
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres et d'éventuellement un co-initiateur puis on la laisse se dérouler,
c) lorsque la réaction de polymérisation est terminée, l'on introduit un ou plusieurs agents émulsifiants de type huile dans eau à une température inférieure à 50°C.

Selon une variante de ce procédé, le milieu réactionnel issu de l'étape b), est concentré par distillation, avant la mise en oeuvre de l'étape c).

Selon une mise en oeuvre préférée du procédé tel que défini précédemment, la réaction de polymérisation est amorcée par un couple oxydoréducteur générateur d'ions hydrogénosulfite (HSO₃⁻), tel que le couple hydroperoxyde de cumène -métabisulfite de sodium (Na₂S₂O₅) ou le couple hydroperoxyde de cumène-chlorure de thionyle (SOCI₂) à une température inférieure ou égale à 10°C, si désiré accompagné d'un agent co-initiateur de polymérisation tel que par exemple l'azo-bis(isobutyronitrile) puis conduite soit de manière quasi adiabatique jusqu'à une température supérieure ou égale à 50°C, soit en contrôlant la température.

L'invention a aussi pour objet, une composition cosmétique, dermopharmaceutique ou pharmaceutique, caractérisée en ce qu'elle comprend au moins un latex inverse auto-inversible tel que défini précédemment.

La composition cosmétique, dermopharmaceutique ou pharmaceutique définie ci-dessus comprend généralement de 0,1 % à 10 % et plus particulièrement entre 0,5% et 5% en poids dudit latex. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après shampooings. Il peut aussi être utilisé en combinaison avec lesdits SEPIGEL II est notamment compatible avec les concentrés décrits et revendiqués dans les publications internationales WO 92/06778, WO 95/04592, W095/13863, WO 98/47610 ou FR 2734 496 ou avec les agents tensioactifs décrits dans WO 93/08204.

Il est également compatible avec le MONTANOV™ 68, le MONTANOV™ 82, le MONTANOV™ 202 ou le SEPIPERL™ N.

Il peut être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrits dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveu ou de la peau telles que celles décrites dans EP 0 603 019. ou encore dans les shampooings ou après shampooings tels que ceux décrits et revendiqués dans WO 92/21316 ou enfin en association avec un homopolymère anionique tels que le CARBOPOL™ pour former des produits de traitement des cheveux comme ceux décrits dans DE 195 23596.

Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249, EP 0576188 ou dans WO 93/07902.

Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611.

Il est aussi compatible avec les acides glycoliques, l'acide lactique, l'acide salicylique, les rétinoïdes, le phénoxy éthanol, les sucres, le glycéraldéhyde, les xanthanes, les acides de fruit, et les divers polyols utilisés dans la fabrication de formulations cosmétiques.

La composition selon l'invention est de façon toute particulière, compatible avec les actifs cosmétiques riches en sel minéraux, par exemple en sel de sodium ou de magnésium. Elle est compatible, notamment avec le SEPICALM™ S, le SEPICONTROL™ A5, le 2-pyrolidone carboxylate de sodium, le PROTEOL™ OAT ou l'AJIDEW™ A100.

L'invention a donc aussi pour objet, l'utilisation d'un latex inverse auto-inversible, tel que défini précédemment, pour préparer une composition cosmétique, dermopharmaceutique ou pharmaceutique.

L'invention a aussi pour objet une composition cosmétique, dermopharmaceutique ou pharmaceutique telle que définie précédemment et contenant jusqu'à 5% en poids d'un ou plusieurs cations métalliques choisis notamment parmi les cations sodium, potassium, magnésium, manganèse ou aluminium.

Les exemples suivants illustrent la présente invention sans toutefois la limiter.

### Exemple comparatif (a)

### Mode opératoire

On charge dans un bêcher sous agitation :
- 200 g d'eau permutée,
- 73,1 g d'acide acrylique glacial,
- 112,1 g d'une solution aqueuse d'hydroxyde de sodium à 48% en poids,
- 278,4 g d'acide 2-méthyl 2-[(1-oxo 2-propényl) amino] 1-propane-sulfonique
- 0,45 g d'une solution commerciale à 40% en poids de diéthylènetriamine pentaacétate de sodium,
- 0,182 g d'une solution commerciale à 50% de méthylène bis(acrylamide)
Le pH de la phase aqueuse est ajusté à 3,5 et la solution est complétée avec de l'eau permutée jusqu'à 682 g.
Parallèlement, on prépare une phase organique en introduisant dans un bêcher successivement et sous agitation :
- 220 g d'isohexadécane,
- 25 g de MONTANE™ 80 VG (oléate de sorbitan commercialisé par la société SEPPIC),
- 0,2g d'azo bis(isobutyronitrile) (AIBN).
La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation, est soumise à un barbotage d'azote important, pour éliminer l'oxygène, puis est refroidie à 5-6°C. On introduit alors 5 ml d'une solution d'isohexadécane contenant 0,42% en poids d'hydroperoxyde de cumène. Après le temps suffisant pour une bonne homogénéisation de la solution, on introduit, pendant 60 minutes, une solution aqueuse de métabisulfite de sodium (0,2 g / 100 ml d'eau) à raison de 0,5ml /minute en laissant monter la température jusqu'à stabilisation de cette dernière. Le milieu réactionnel est maintenu à cette température pendant 90 minutes, puis L'ensemble est refroidi jusqu'environ 35°C et on ajoute enfin 50g de MONTANOX™80, pour obtenir l'émulsion désirée.

### Evaluation des propriétés du latex obtenu

Viscosité à 25°C du latex à 3% dans l'eau (Brookfield RVT, Mobile 6, vitesse 5) η = 78 000 mPas ;
Viscosité à 25°C du latex à 3% dans l'eau + 0,1% NaCI (Brookfield RVT, Mobile 6, vitesse 5) : η = 20000 mPas ;

### Exemple comparatif (b)

### Mode opératoire

On charge dans un bécher sous agitation :
- 220 g d'eau permutée,
- 138,1 g d'une solution aqueuse d'hydroxyde de sodium à 48% en poids,
- 343,5 g d'acide 2-méthyl -[(1-oxo 2-propényl) amino] 1-propane-sulfonique,
- 0,45 g d'une solution commerciale à 40% en poids de diéthylènetriamine pentaacétate de sodium,
- 0,22 g de méthylène bis(acrylamide)
Le pH de la phase aqueuse est ajusté à 3,5 et la solution est complétée avec de l'eau permutée jusqu'à 707 g.
Parallèlement, on prépare une phase organique en introduisant dans un bécher successivement et sous agitation :
- 220 g d'isohexadécane,
- 22 g de MONTANE™ 80 VG,
- 0,2g d'azo bis(isobutyronitrile) (AIBN).
La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation, est soumise à un barbotage d'azote important, pour éliminer l'oxygène, puis est refroidie à 5-6°C. On introduit alors 5ml d'une solution d'isohexadécane contenant 0,42% en poids d'hydroperoxyde de cumène. Après le temps suffisant pour une bonne homogénéisation de la solution, on introduit, pendant 60 minutes, une solution aqueuse de métabisulfite de sodium (0,4g / 100 ml d'eau) à raison de 0,5ml / minute en laissant monter la température jusqu'à la température de polymérisation. Le milieu réactionnel est maintenu à cette température pendant 90 minutes, puis L'ensemble est refroidi jusque environ 35°C et on ajoute enfin 30g de MONTANOX™80, pour obtenir l'émulsion désirée.

### Evaluation des propriétés du latex obtenu

Viscosité à 25°C du latex à 3% dans l'eau (Brookfield RVT, Mobile 6, vitesse 5) : η = 93 800 mPas ;
Viscosité à 25°C du latex à 3% dans l'eau + 0,1% NaCI (Brookfield RVT, Mobile 6, vitesse 5) : η = 20 200 mPas ;

### Exemple comparatif (c)

### Mode opératoire

On charge dans un bêcher sous agitation :
- 250 g d'eau permutée,
- 250 g d'acide acrylique glacial,
- 170 g environ d'une solution d'ammoniaque à 30% en poids, de façon à ramener le pH à 5,5,
- 0,45 g d'une solution commerciale à 40% en poids de diéthylènetriamine pentaacétate de sodium,
- 2,31 g d'une solution commerciale à 50% de diallyloxyacétate de sodium, et la solution est complétée avec de l'eau permutée jusqu'à 682 g.
Parallèlement, on prépare une phase organique en introduisant dans un bécher successivement et sous agitation :
- 220 g d'isohexadécane,
- 30 g de MONTANE™ 80 VG,
- 0,2g d'azo bis(isobutyronitrile) (AIBN).
La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation, est soumise à un barbotage d'azote important, pour éliminer l'oxygène, puis est refroidie à 5-6°C. On introduit alors 0,16 g de peroxydisulfate de sodium dilué dans 20 g d'eau. Après le temps suffisant pour une bonne homogénéisation de la solution, on introduit, pendant 60 minutes, une solution aqueuse de métabisulfite de sodium (0,2 g / 100 ml d'eau) à raison de 0,5 ml /minute en laissant monter la température jusqu'à la température de polymérisation. Le milieu réactionnel est maintenu à cette température pendant 90 minutes, puis L'ensemble est refroidi jusque environ 35°C et on ajoute enfin 50g de SIMULSOL™ OL50 pour obtenir l'émulsion désirée.

### Evaluation des propriétés du latex obtenu

Viscosité à 25°C du latex à 3% dans l'eau (Brookfield RVT, Mobile 6, vitesse 5) : η = 93 000 mPas ;
Viscosité à 25°C du latex à 3% dans l'eau + 0,1% NaCI (Brookfield RVT, Mobile 6, vitesse 5) : η = 20 200 mPas ;

### Exemple comparatif (d)

### Mode opératoire

On charge dans un bêcher sous agitation :
- 250 g d'eau permutée,
- 250 g d'acide acrylique glacial,
- 149,6 g de monoéthanolamine, de manière à amener le pH de la solution à 5,5,
- 0,45 g d'une solution commerciale à 40% de diéthylènetriamine pentaacétate de sodium,
- 3, 4 g d'une solution commerciale à 50% de diallyloxyacétate de sodium. On complète avec de l'eau permutée jusqu'à 682 g.
Parallèlement, on prépare une phase organique en introduisant dans un bécher successivement et sous agitation ::
- 220g d'isohexadécane,
- 30g de MONTANE™ 80 VG (oléate de sorbitan commercialisé par la société SEPPIC),
- 0,2g d'AIBN.
La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation, est soumise à un barbotage d'azote important, pour éliminer l'oxygène, puis est refroidie à 5-6°C. On introduit alors 0,039 g de d'hydro peroxyde de cumène en solution dans de l'isohexadécane. Après le temps suffisant pour une bonne homogénéisation de la solution, on introduit, pendant 60 minutes, une solution aqueuse de métabisulfite de sodium (0,4 g /100 ml d'eau) à raison de 0,5ml /minute en laissant monter la température jusqu'à la température de polymérisation. Le milieu réactionnel est maintenu à cette température pendant 90 minutes, puis L'ensemble est refroidi jusque environ 35°C et on ajoute enfin 50 g de SIMULSOL™ OL 50, pour obtenir l'émulsion désirée.

### Evaluation des propriétés du latex obtenu

Viscosité à 25°C du latex à 3% dans l'eau (Brookfield RVT, Mobile 6, vitesse 5) : η = 114 000 mPas ;
Viscosité à 25°C du latex à 3% dans l'eau + 0,1% NaCI (Brookfield RVT, Mobile 6, vitesse 5) : η = 35 000 mPas ;

### Exemple 1 : Latex selon l'invention à base de copolymère AMPS/acide acrylique (salifié sous forme de sel de sodium)

En procédant comme à l'exemple comparatif (a), mais en introduisant 54, 6g de SIMULSOL™ SL 10, au lieu du MONTANOX™80, on obtient un latex ayant les propriétés viscosimétriques suivantes :
Viscosité à 25°C du latex à 3% dans l'eau (Brookfield RVT, Mobile 6, vitesse 5) : η = 77 800 mPas ;
Viscosité à 25°C du latex à 3% dans l'eau + 0,1% NaCI (Brookfield RVT, Mobile 6, vitesse 5) : η = 30 000 mPas ;

### Exemple 2 : Latex selon l'invention à base d'homopolymère d'AMPS (salifié sous forme de sel de sodium)

En procédant comme à l'exemple comparatif (b), mais en introduisant 54,6 g de SIMULSOL™ SL 10, au lieu du MONTANOX™80, on obtient un latex ayant des propriétés viscosimétriques suivantes :
Viscosité à 25°C du latex à 3% dans l'eau (Brookfield RVT, Mobile 6, vitesse 5) : η = 92 400 mPas ;
Viscosité à 25°C du latex à 3% dans l'eau + 0,1% NaCI (Brookfield RVT, Mobile 6, vitesse 5) : η = 30 000 mPas ;

### Exemple 3 : Latex selon l'invention à base d'homopolymère d'acide acrylique (salifié sous forme de sel d'ammonium)

En procédant comme à l'exemple comparatif (c), mais en introduisant 50g de SIMULSOL™ SL 10, au lieu du SIMULSOL™ OL50, on obtient un latex ayant des propriétés viscosimétriques suivantes :
Viscosité à 25°C du latex à 3% dans l'eau (Brookfield RVT, Mobile 6, vitesse 5) : η = 93 000 mPas ;
Viscosité à 25°C du latex à 3% dans l'eau + 0,1% NaCI (Brookfield RVT, Mobile 6, vitesse 5) : η = 30 000 mPas ;

### Exemple 4 : Latex selon l'invention à base d'homopolymère d'acide acrylique [salifié sous forme de sel de [N-(2-hydroxy éthyl)ammonium]].

En procédant comme à l'exemple comparatif (d), mais en introduisant 54,6 g de SIMULSOL™ SL10, au lieu du SIMULSOL™ OL 50, on obtient un latex ayant Les propriétés viscosimétriques suivantes :
Viscosité à 25°C du latex à 3% dans l'eau (Brookfield RVT, Mobile 6, vitesse 5) : η = 106 400 mPas ;
Viscosité à 25°C du latex à 3% dans l'eau + 0,1% NaCI (Brookfield RVT, Mobile 6, vitesse 5) : η = 45 800 mPas ;

### Exemple 5 : Latex selon l'invention à base d'homopolymère d'acide acrylique [salifié sous forme de sel de [N-(2-hydroxy éthyl) ammonium]].

En procédant comme à l'exemple comparatif (d), mais en introduisant 75,2g de SIMULSOL™ SL 26, au lieu du SIMULSOL™ OL 50, on obtient un latex ayant Les propriétés viscosimétriques suivantes :
Viscosité à 25°C du latex à 3% dans l'eau (Brookfield RVT, Mobile 6, vitesse 5) : η = 82 000 mPas ;
Viscosité à 25°C du latex à 3% dans l'eau + 0,1% NaCI (Brookfield RVT, Mobile 6, vitesse 5) : η = 45 200 mPas ;

### Analyse des résultats

Les viscosités, mesurées à25 °C (Brookfield RVT, Mobile 6, vitesse 5 ; en mPas), sont consignées dans le tableau suivant ; la comparaison entre les pertes de viscosité constatées [(η₁-η)/_{η} = Δ_{η}/_{η}], pour les latex des exemples comparatifs (a) à (d), et celles constatées pour les latex des exemples 1 à 5, permet de démontrer que l'amélioration de la tenue au sel des latex selon l'invention, est inhérente à la présence d'alkyl polyglycosides de formule (I).

| Exemple N° | Viscosité η du latex à 3% dans l'eau | Viscosité η₁ du latex à 3% dans l'eau + 0,1% NaCI | Δ_{η}/ _{η} |
|---|---|---|---|
| a | 78000 | 20000 | -74,36% |
| 1 | 77800 | 30000 | -61,44% |
| b | 93800 | 20200 | -78,50% |
| 2 | 92400 | 30000 | -67,53% |
| c | 93000 | 20200 | -78,28% |
| 3 | 93000 | 30000 | -67,74% |
| d | 114000 | 35000 | -69,30% |
| 4 | 106400 | 45800 | -56,95% |
| 5 | 82000 | 45200 | -44,89% |

### Exemples de compositions cosmétiques

On met en oeuvre indifféremment l'un des latex préparés aux exemples 1à 5 dans les compositions cosmétiques suivantes :

### Exemple 6 : Crème de soin

| | |
|---|---|
| Cyclométhicone | 10% |
| Composition 1 | 0,8% |
| MONTANOV™68 | 2% |
| Alcool stéarylique | 1,5% |
| conservateur | 0,65% |
| Lysine | 0,025% |
| EDTA (sel disodique) | 0,05% |
| Gomme de xanthane | 0,2% |
| Glycérine | 3 % |
| Eau | q.s.p. 100% |

### Exemple 7 : Crème de soin

| | |
|---|---|
| Cyclométhicone | 10% |
| Composition 1 | 0,8% |
| MONTANOV™ 68 | 2% |
| Perfluoropolymethylisopropylether | 0,5% |
| Alcool stéarylique | 1,5% |
| conservateur | 0,65% |
| Lysine | 0,025% |
| EDTA (sel disodique) | 0,05% |
| PEMULEN™ TR | 0,2% |
| Glycérine | 3% |
| Eau | q.s.p. 100% |

### Exemple 8 : Baume après-rasage

### FORMULE

| | |
|---|---|
| A Composition 1 | 1,5% |
| Eau | q.s.p 100% |
| MICROPEARL™ M 100 | 5,0% |
| SEPICIDE™ CI | 0,50% |
| Parfum | 0,20% |
| Ethanol 95° | 10,0% |

### MODE OPERATOIRE

Ajouter B dans A.

### Exemple 9 : Emulsion satinée pour le corps

### FORMULE

| | | |
|---|---|---|
| A | SIMULSOL™ 165 | 5,0% |
| | LANOL™ 1688 | 8,50% |
| | Beurre de Karité | 2% |
| | Huile de paraffine | 6,5% |
| | LANOL™ 14M | 3% |
| | LANOL™ S | 0,6% |
| B | eau | 66,2% |
| C | MICROPEARL™ M 100 | 5% |
| D | Composition 1 | 3% |
| E | SEPICIDE™ CI | 0,3% |
| | SEPICIDE™ HB | 0,5% |
| | MONTEINE™ CA | 1% |
| | Parfum | 0,20% |
| | Acétate de vitamine E | 0,20% |
| | Sodium pyrrolidinonecarboxylate | 1% (agent hydratant) |

### MODE OPERATOIRE

Ajouter C dans B, émulsionner B dans A à 70°C, puis ajouter D à 60°C puis E à 30°C

### Exemple 10 : Lait corporel

### FORMULE

| | | |
|---|---|---|
| A | SIMULSOL™ 165 | 5,0% |
| | LANOL™ 1688 | 12,0% |
| | LANOL™ 14M | 2,0% |
| | Alcool cétylique | 0,3% |
| | SCHERCEMOL™ OP | 3% |
| B | eau | q.s.p. 100% |
| C | Composition 1 | 0,35% |
| D | SEPICIDE™ CI | 0,2% |
| | SEPICIDE™ HB | 0,5% |
| | Parfum | 0,20% |

### MODE OPERATOIRE

Emulsionner B dans A vers 75°C ; ajouter C vers 60°C, puis D vers 30°C

### Exemple 11 : Crème H/E

### FORMULE

| | | |
|---|---|---|
| A | SIMULSOL™ 165 | 5,0% |
| | LANOL™ 1688 | 20,0% |
| | LANOL™ P | 1,0% (additif à effet stabilisant) |
| B | eau | q.s.p. 100% |
| C | Composition 1 | 2,50% |
| D | SEPICIDE™ CI | 0,20% |
| | SEPICIDE™ HB | 0,30% |

### MODE OPERATOIRE

Introduire B dans A vers 75°C ; ajouter C vers 60°C, puis D vers 45°C

### Exemple 12 : gel solaire non gras

### FORMULE

| | | |
|---|---|---|
| A | Composition 1 | 3,00% |
| | Eau | 30% |
| B | SEPICIDE™ CI | 0,20% |
| | SEPICIDE™ HB | 0,30% |
| | Parfum | 0,10% |
| C | colorant | q.s. |
| | eau | 30% |
| D | MICROPEARL™ M 100 | 3,00% |
| | Eau | q.s.p 100% |
| E | huile de silicone | 2,0% |
| | PARSOL™ MCX | 5,00% |

### MODE OPERATOIRE

Introduire B dans A; ajouter C, puis D, puis E.

### Exemple13 : Lait solaire

### FORMULE

| | | |
|---|---|---|
| A | SEPIPERL™ N | 3,0% |
| | Huile de sésame | 5,0% |
| | PARSOL™ MCX | 5,0% |
| | Carraghénane λ | 0,10% |
| B | eau | q.s.p. 100% |
| C | Composition 1 | 0,80% |
| D | Parfum | q. s. |
| | Conservateur | q. s. |

### MODE OPERATOIRE

Emulsionner B dans A à 75°C puis ajouter C vers 60°C, puis D vers 30°C et ajuster le pH si nécessaire

### Exemple 14 : Gel de massage

### FORMULE

| | | |
|---|---|---|
| A | Composition 1 | 3,5% |
| | Eau | 20,0% |
| B | colorant | 2 gouttes/100g |
| | Eau | q. s. |
| C | alcool | 10% |
| | Menthol | 0,10% |
| D | huile de silicone | 5,0% |

### MODE OPERATOIRE

Ajouter B dans A ; puis ajouter au mélange, C puis D

### Exemple 15 : gel soin de massage

### FORMULE

| | | |
|---|---|---|
| A | Composition 1 | 3,00% |
| | Eau | 30% |
| B | SEPICIDE™ CI | 0,20% |
| | SEPICIDE™ HB | 0,30% |
| | Parfum | 0,05% |
| C | colorant | q. s. |
| | Eau | q. s. p. 100% |
| D | MICROPEARL™ SQL | 5,00% |
| | LANOL™ 1688 | 2% |

### MODE OPERATOIRE

Préparer A ; additionner B, puis C, puis D.

### Exemple 16 : Gel coup d'éclat

### FORMULE

| | | |
|---|---|---|
| A | Composition 1 | 4% |
| | Eau | 30% |
| B | ELASTINE HPM | 5,0% |
| C | MICROPEARL™ M 100 | 3% |
| | Eau | 5% |
| D | SEPICIDE™ CI | 0,2% |
| | SEPICIDE™ HB | 0,3% |
| | Parfum | 0,06% |
| Sodium pyrrolidinonecarboxylate 50% | | 1% |
| Eau | | q. s. p. 100% |

### MODE OPERATOIRE

Préparer A ; additionner B, puis C, puis D.

### Exemple 17 : Lait corporel

### FORMULE

| | | |
|---|---|---|
| A | SEPIPERL™ N | 3,0% |
| | Triheptonate de glycérol | 10,0% |
| B | eau | q.s.p.100% |
| C | Composition 1 | 1,0% |
| D | parfum | q. s. |
| | Conservateur | q. s. |

### MODE OPERATOIRE

Fondre A à environ 75°C . Emulsionner B dans A à 75°C puis ajouter C vers 60°C, puis D .

### Exemple 18 : Emulsion démaquillante à l'huile d'amandes douces

### FORMULE

| | |
|---|---|
| MONTANOV™ 68 | 5% |
| huile d'amandes douces | 5% |
| eau | q.s.p.100% |
| Composition 1 | 0,3% |
| glycérine | 5% |
| conservateur | 0,2% |
| parfum | 03% |

### Exemple 19 : Crème hydratante pour peaux grasses

### FORMULE

| | |
|---|---|
| MONTANOV™ 68 | 5% |
| Cétylstéaryloctanoate | 8% |
| octyl palmitate | 2% |
| eau : | q.s.p.100% |
| Composition 1 | 0,6% |
| MICROPEARL™ M100 | 3,0% |
| Mucopolysaccharides | 5% |
| SEPICIDE™ HB | 0,8% |
| Parfum | 0 ,3% |

### Exemple 20 : Baume après-rasage apaisant sans alcool

### FORMULE

| | |
|---|---|
| mélange de lauryl aminoacides | 0,1% à 5% |
| aspartate de magnésium et de potassium | 0,002% à 0,5% |
| LANOL™ 99 | 2% |
| huile d'amandes douces | 0,5% |
| eau | q.s.p.100% |
| Composition 1 | 3% |
| SEPICIDE™ HB | 0,3% |
| SEPICIDE™ CI | 0,2% |
| Parfum | 0,4% |

### Exemple 21 : Crème aux AHA pour peaux sensibles

### FORMULE

| | |
|---|---|
| mélange de lauryl aminoacides | 0,1% à 5% |
| aspartate de magnésium et de potassium | 0,002% à 0,5% |
| LANOL™ 99 | 2% |
| MONTANOV™ 68 | 5,0% |
| Eau | q.s.p.100% |
| Composition 1 | 1,50% |
| acide gluconique | 1,50% |
| triéthanolamine | 0,9% |
| SEPICIDE™ HB | 0,3% |
| SEPICIDE™ CI | 0,2% |
| Parfum | 0,4% |

### Exemple 22 : Soin apaisant après soleil

### FORMULE

| | |
|---|---|
| mélange de lauryl aminoacides | 0,1% à 5% |
| aspartate de magnésium et de potassium | 0,002% à 0,5% |
| LANOL™ 99 | 10,0% |
| Eau | q.s.p.100% |
| Composition 1 | 2,50% |
| SEPICIDE™ HB | 0,3% |
| SEPICIDE™ CI | 0,2% |
| Parfum | 0,4% |
| Colorant | 0,03% |

### Exemple 23 : Lait démaquillant

### FORMULE

| | |
|---|---|
| SEPIPERL™ N | 3% |
| PRIMOL™ 352 | 8,0% |
| huile d'amandes douces | 2 % |
| eau | q.s.p.100% |
| Composition 1 | 0,8% |
| conservateur | 0,2% |

### Exemple 24 : Lait corporel

### FORMULE

| | |
|---|---|
| SEPIPERL™ N | 3,5% |
| LANOL™ 37T | 8,0% |
| SOLAGUM™ L | 0,05% |
| Eau | q.s.p.100% |
| Benzophénone | 2,0% |
| diméthicone 350cPs | 0,05% |
| Composition 1 | 0,8% |
| conservateur | 0,2% |
| parfum | 0,4% |

### Exemple 25 : Fond de teint fluide

### FORMULE

| | |
|---|---|
| SIMULSOL™ 165 | 5,0% |
| LANOL™ 84D | 8,0% |
| LANOL™ 99 | 5,0% |
| Eau | q.s.p.100% |
| pigments et charges minérales | 10,0% |
| Composition 1 | 1,2% |
| conservateur | 0,2% |
| parfum | 0,4% |

### Exemple 26 : Lait solaire

### FORMULE

| | |
|---|---|
| SEPIPERL™ N | 3,5% |
| LANOL™ 37T | 10,0% |
| PARSOL NOX™ | 5,0% |
| EUSOLEX™ 4360 | 2,0% |
| Eau | q.s.p.100% |
| Composition 1 | 1,8% |
| conservateur | 0,2% |
| parfum | 0,4% |

### Exemple 27 : Gel contour des yeux

### FORMULE

| | |
|---|---|
| Composition 1 | 2,0% |
| Parfum | 0,06% |
| Sodium pyrrolidinonecarboxylate | 0,2% |
| DOW CORNING™ 245 Fluid | 2,0% |
| Eau | q. s. p. 100% |

### Exemple 28 : Composition de soin non rincée

### FORMULE

| | |
|---|---|
| Composition 1 | 1,5% |
| Parfum | q. s |
| Conservateur | q. s. |
| DOW CORNING™ X2 8360 | 5,0% |
| DOW CORNING™ Q2 1401 | 15,% |
| Eau | q.s.p. 100% |

### Exemple 29 : Gel amincissant

| | |
|---|---|
| Composition 1 | 5 % |
| Ethanol | 30 % |
| Menthol | 0,1 % |
| Caféine | 2,5 % |
| extrait de ruscus | 2 % |
| extrait de lierre | 2 % |
| SEPICIDE™ HP | 1 % |
| Eau | q. s. p. 100 % |

### Exemple 30 : Baume après-rasage apaisant sans alcool

### FORMULE

| | | |
|---|---|---|
| A | LIPACIDE™ PVB | 1,0% |
| | LANOL™ 99 | 2,0% |
| | Huile d'amandes douces | 0,5% |
| B | Composition 1 | 3,5% |
| C | eau | q.s.p.100% |
| D | parfum | 0,4% |
| | SEPICIDE™ HB | 0,4% |
| | SEPICIDE™ CI | 0,2% |

### Exemple 31 : Gel rafraîchissant après-rasage

### FORMULE

| | | |
|---|---|---|
| A | LIPACIDE™ PVB | 0,5% |
| | LANOL™ 99 | 5,0% |
| | Composition 1 | 2,5% |
| B | eau | q.s.p.100% |
| C | MICROPEARL™ LM | 0,5% |
| | Parfum | 0,2% |
| | SEPICIDE™ HB | 0,3% |
| | SEPICIDE™ CI | 0,2% |

### Exemple 32 : Soin pour les peaux grasses

### FORMULE

| | | |
|---|---|---|
| A | MICROPEARL™ M310 | 1,0% |
| | Composition 1 | 5,0% |
| | Isononanoate d'octyle | 4.0% |
| B | eau | q.s.p.100% |
| C | SEPICONTROL™ A5 | 4,0% |
| | Parfum | 0,1% |
| | SEPICIDE™ HB | 0,3% |
| | SEPICIDE™ CI | 0,2% |
| D | CAPIGEL™ 98 | 0,5% |
| | Eau : | 10% |

### Exemple 33 : Crème aux AHA

### FORMULE

| | | |
|---|---|---|
| A | MONTANOV™ 68 | 5,0% |
| | LIPACIDE™ PVB | 1,05% |
| | LANOL™ 99 | 10,0% |
| B | eau | q.s.p.100% |
| | Acide gluconique | 1,5% |
| | Triéthanolamine) | 0,9% |
| C | Composition 1 | 1,5% |
| D | parfum | 0,4% |
| | SEPICIDE™ HB | 0,2% |
| | SEPICIDE™ Cl | 0,4% |

### Exemple 34 : Autobronzant non gras pour visage et corps

### FORMULE

| | | |
|---|---|---|
| A | LANOL™ 2681 | 3,0% |
| | Composition 1 | 2,5% |
| B | eau | q.s.p.100% |
| | Dihydroxyacétone | 3,0% |
| C | parfum | 0,2% |
| | SEPICIDE™ HB | 0,8% |
| | NaOH (hydroxyde de sodium) | qs pH = 5 |

### Exemple 35 : Lait solaire au monoï de Tahiti

### FORMULE

| | | |
|---|---|---|
| A | Monoï de Tahiti | 10% |
| | LIPACIDE™ PVB | 0,5% |
| | Composition 1 | 2,2% |
| B | eau | q.s.p.100% |
| C | parfum | 0,1% |
| | SEPICIDE™ HB | 0,3% |
| | SEPICIDE™ CI | 0,1% |
| | Méthoxycinnamate d'octyle | 4,0% |

### Exemple 36 : Soin solaire pour le visage

### FORMULE

| | | |
|---|---|---|
| A | Cyclométhicone et diméthiconol | 4,0% |
| | Composition 1 | 3,5% |
| B | eau | q.s.p.100% |
| C | parfum | 0,1% |
| | SEPICIDE™ HB | 0,3% |
| | SEPICIDE™ CI | 0,21% |
| | Méthoxycinnamate d'octyle | 5,0% |
| | Micatitane | 2,0% |
| | Acide lactique | q.s.p. pH = 6,5 |

### Exemple 37 : Emulsion bronzante sans soleil

### FORMULE

| | | |
|---|---|---|
| A | LANOL™ 99 | 15% |
| | MONTANOV™ 68 | 5,0% |
| | Paraméthoxycinnamate d'octyle | :3,0% |
| B | eau | q.s.p.100% |
| | Dihydroxyacétone | 5,0% |
| | Phosphate monosodique | 0,2% |
| C | Composition 1 | 0,5% |
| D | parfum | 0,3% |
| | SEPICIDE™ HB | 0,8% |
| | NaOH | q.s. pH=5. |

Les noms commerciaux cités ci-dessus, ont les définitions suivantes :
Le MONTANOV™ 68 (cétéaryl glucoside), est une composition auto-émulsionnable telle que décrite dans WO 92/06778, commercialisée par la société SEPPIC.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL ® S sont des facteurs de consistance commercialisés par la société SEPPIC.
Le SEPICIDE™ HB , qui est un mélange de phénoxyéthanol, de méthyl paraben, d'éthylparaben, de propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le PARSOL™ MCX est de l'octyl paraméthoxycinnamate; commercialisé par la société GIVAUDAN.
Le SEPIPERL™ N est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage; il est commercialisé par la société MATSUMO.
Le LANOL™ 99 est de l'isononyl isononanoate commercialisé par la société SEPPIC.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société ESSO.
Le LANOL™ 84D est du malate de dioctyle commercialisé par la société SEPPIC.
Le PARSOL NOX™ est un filtre solaire commercialisé par la société GIVAUDAN.
I' EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB est un hydrolysat de protéines de blé palmitoylé, commercialisée par la société SEPPIC.
Le MICROPEARL™ LM est un mélange de squalane, de poly(méthylméthacrylate) et de Menthol, commercialisé par la société SEPPIC.
Le SEPICONTROL™ A5 est un mélange capryloyl glycine, sarcosine, extrait de cinnamon zylanicum, commercialisé par la société SEPPIC, tel que ceux décrits dans la publication internationale de brevet numéro WO 99/00109
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 40% en poids, d'un polyélectrolyte linéaire, branché ou réticulé, caractérisé en ce que ledit polyélectrolyte est, soit un homopolymère à base d'un monomère possédant une fonction acide fort partiellement ou totalement salifiée, soit un homopolymère à base d'un monomère possédant une fonction acide faible partiellement ou totalement salifiée, soit un homopolymère à base d'un monomère cationique, soit un copolymère à base d'au moins un monomère possédant une fonction acide fort partiellement ou totalement salifiée, copolymérisé, ou bien avec au moins un monomère possédant une fonction acide faible partiellement ou totalement salifiée, ou bien avec au moins un monomère neutre, soit un copolymère à base d'un monomère cationique, copolymérisé, avec au moins un monomère neutre, et caractérisé en ce que l'un au moins des agents émulsifiants de type huile dans eau est un composé de formule (I) :
R₁-O-[CH(R₂)-CH₂-O]ₙ-(G)ₓ-H (I)
dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal soit à zéro soit à un nombre entier compris entre 1 et 30.

2. Composition telle que définie à la revendication 1, dans laquelle l'agent émulsifiant du type eau dans huile, est choisi parmi les esters de sorbitan, comme le monooléate de sorbitan ou l'isostéarate de sorbitan.

3. Composition telle que définie à l'une des revendications 1 ou 2, dans laquelle l'agent émulsifiant de type huile dans eau est un composé de formule (I) :
R₁-O-[CH(R₂)-CH₂-O]ₙ-(G)ₓ-H (I)
ou un mélange de composés de formule (I).

4. Composition telle que définie à l'une quelconque des revendications 1 à 3, dans laquelle on désigne pour R₁ dans la formule (I), les radicaux alkyles ou les radicaux alkènyle, plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés..

5. Composition telle que définie à l'une quelconque des revendications 1 à 4, dans laquelle, dans la formule (I), x est compris entre 1 et 3, notamment entre 1,05 et 2,5, tout particulièrement entre 1,1 et 2,0 et de préférence inférieur ou égal à 1,5.

6. Composition telle que définie à l'une quelconque des revendications 1 à 5, dans laquelle on désigne pour G dans la formule (I), le reste du glucose.

7. Composition telle que définie à l'une quelconque des revendications 1 à 5, dans laquelle on désigne pour G dans la formule (I), le reste du xylose.

8. Composition telle que définie à l'une quelconque des revendications 1 à 7, dans laquelle, dans la formule (I), n est égal à 0.

9. Composition telle que définie à l'une quelconque des revendications 1 à 8, dans laquelle on désigne pour R₁ dans la formule (I), un radical comportant de 8 à 18 atomes de carbone et plus particulièrement, un des radicaux octyle, décyle, undécyle, dodécyle, tétradécyle ou hexadécyle, lesdits radicaux étant linéaires ou ramifiés.

10. Composition telle que définie à l'une quelconque des revendications 1 à 9, caractérisée en ce que le polyélectrolyte est réticulé et/ou branché par un agent de réticulation et/ou un agent ramification choisi parmi les composés diéthyléniques ou polyéthyléniques, et tout particulièrement parmi l'acide diallyloxyacétique ou un des sels et notamment son sel de sodium, le triallylamine, le triméthylol propanetriacrylate, le diméthacrylate d'éthylèneglycol, le diacrylate de diéthylène glycol, le diallylurée ou le méthylène bis(acrylamide).

11. Composition telle que définie à la revendication 10, caractérisée en ce que l'agent de réticulation et/ou de ramification est dans la proportion molaire exprimée par rapport aux monomères mis en oeuvre, de 0,005% à 1%, notamment de 0,01% à 0,2% et plus particulièrement de 0,01% à 0,2%.

12. Composition telle que définie à l'une quelconque des revendications 1 à 11, caractérisée en ce que ledit polyélectrolyte est, soit un homopolymère à base d'un monomère possédant une fonction acide fort partiellement ou totalement salifiée, soit un copolymère à base d'au moins un monomère possédant une fonction acide fort partiellement ou totalement salifiée, copolymérisé, ou bien avec au moins un monomère possédant une fonction acide faible partiellement ou totalement salifiée, ou bien avec au moins un monomère neutre.

13. Composition telle que définie à la revendication 11, caractérisée en ce que le monomère à fonction acide fort est l'acide 2-méthyl 2-[(1-oxo 2-propènyl) amino] 1-propane-sulfonique, partiellement ou totalement salifié, sous forme, de sel de métal alcalin, tel que le sel de sodium ou le sel de potassium, de sel d'ammonium (NH₄⁺) ou de sel d'aminoalcool tel que le sel de monoéthanolamine (HOCH₂CH₂NH₃⁺).

14. Composition telle que définie à l'une quelconque des revendications 1 à 11, caractérisée en ce que ledit polyélectrolyte est un homopolymère à base d'un monomère possédant une fonction acide faible partiellement ou totalement salifiée.

15. Composition telle que définie à l'une des revendications 12 ou 14, caractérisée en ce que le monomère à fonction acide faible est choisi parmi l'acide acrylique, l'acide méthacrylique, l'acide itaconique ou l'acide maléique partiellement ou totalement salifié sous forme, de sel de métal alcalin, tel que le sel de sodium ou le sel de potassium, de sel d'ammonium (NH₄⁺) ou de sel d'aminoalcool tel que le sel de monoéthanolamine (HOCH₂CH₂NH₃⁺).

16. Composition telle que définie à la revendication 12, caractérisée en ce que le monomère neutre est choisi parmi l'acrylamide, le méthacrylamide, le vinyl pyrrolidone, l'acrylate de (2-hydroxy éthyle), l'acrylate de (2,3-dihydroxy propyle), le méthacrylate de (2-hydroxy éthyle) ou le méthacrylate de (2,3-dihydroxy propyle), ou un dérivé éthoxylé de poids moléculaire compris entre 400 et 1000, de chacun de ces esters hydroxylés décrits ci-dessus.

17. Composition telle que définie à l'une des revendications 1 à 16, dans laquelle le polyélectrolyte anionique est soit un homopolymère de l'acide 2-méthyl 2-[(1-oxo 2-propènyl) amino] 1-propanesulfonique (AMPS) partiellement ou totalement salifié sous forme de sel de sodium, de sel d'ammonium, ou de sel de monoéthanolamine, soit un homopolymère de l'acide acrylique partiellement ou totalement salifié sous forme de sel de sodium, de sel d'ammonium, ou de sel de monoéthanolamine, soit un copolymère comportant en proportion molaire de 30% à 50% d'acide 2-méthyl 2-[(1-oxo 2-propènyl) amino] 1-propanesulfonique partiellement ou totalement salifié sous forme de sel de sodium, de sel d'ammonium, ou de sel de monoéthanolamine et de 50% à 70% d'acrylamide, soit un copolymère comportant en proportion molaire de 60% à 90% d'acide 2-méthyl 2-[(1-oxo 2-propènyl) amino] 1-propanesulfonique partiellement ou totalement salifié sous forme de sel de sodium, de sel d'ammonium, ou de sel de monoéthanolamine et de 10% à 40% d'acrylate de (2-hydroxy éthyle) ou soit un copolymère comportant en proportion molaire de 30% à 90% et plus particulièrement de 30 à 45% d'acide 2-méthyl 2-[(1-oxo 2-propènyl) amino] 1-propanesulfonique partiellement ou totalement salifié sous forme de sel de sodium, de sel d'ammonium, ou de sel de monoéthanolamine et d'acide acrylique partiellement ou totalement salifié sous forme de sel de sodium, de sel d'ammonium, ou de sel de monoéthanolamine.

18. Composition telle que définie à l'une quelconque des revendications 1 à 17, caractérisée en ce qu'elle contient de 2,5% à 15% en poids, et de préférence de 4% à 9% en poids, d'agents émulsifiants, parmi lesquels de 20% à 50%, notamment de 25% à 40% du poids total des agents émulsifiants présents sont du type eau dans huile (E/H) et dans laquelle de 80% à 50%, notamment de 75% à 60%, du poids total des agents émulsifiants, sont du type huile dans eau (H/E).

19. Composition telle que définie à l'une quelconque des revendications 1 à 18 caractérisée en ce que la phase huile représente de 15% à 50%, de préférence de 20% à 25%, de son poids total.

20. Composition telle que définie à l'une quelconque des revendications 1 à 19, dans laquelle la phase huile consiste en de l'isohexadécane ou en une huile blanche minérale.

21. Composition telle que définie à l'une quelconque des revendications 1 à 20, contenant entre 5% et 60% en poids, d'eau et plus particulièrement entre20 % et 50 % en poids d'eau.

22. Composition telle que définie à l'une quelconque des revendications 1 à 21, caractérisé en ce qu'elle contient en outre un ou plusieurs additifs choisis notamment parmi les agents complexant, des agents de transfert ou des agents limiteurs de chaîne.

23. Procédé de préparation de la composition, telle que définie à l'une quelconque des revendications 1 à 22, caractérisé en ce que :
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres et d'éventuellement un co-initiateur puis on la laisse se dérouler,
c) lorsque la réaction de polymérisation est terminée, l'on introduit un ou plusieurs agents émulsifiants de type huile dans eau à une température inférieure à 50°C.

24. Variante du procédé, tel que défini à la revendication 23, caractérisée en ce que le milieu réactionnel issu de l'étape b), est concentré par distillation, avant la mise en oeuvre de l'étape c).

25. Procédé, tel que défini à l'une des revendications 22 ou 23, caractérisé en ce que la réaction de polymérisation est amorcée par un couple oxydoréducteur générateur d'ions hydrogénosulfite (HSO₃⁻), tel que le couple hydroperoxyde de cumène - métabisulfite de sodium (Na₂S₂O₅), ou le couple hydroperoxyde de cumène - chlorure de thionyle (SOCI₂) à une température inférieure ou égale à 10°C, si désiré accompagné d'un agent co-initiateur de polymérisation tel que par exemple l'azo-bis(isobutyronitrile) puis conduite de manière quasi adiabatique jusqu'à une température supérieure ou égale à 50°C.

26. Utilisation de la composition telle que définie à l'une des revendications 1 à 22, pour préparer une composition cosmétique, dermopharmaceutique ou pharmaceutique.

27. Composition cosmétique, dermopharmaceutique ou pharmaceutique comprenant de 0,1% à 10% en poids d'un latex inverse auto-inversible tel que défini à l'une des revendications 1 à 22.

28. Composition cosmétique, dermopharmaceutique ou pharmaceutique telle que définie à la revendication 27 comprenant jusqu'à 5% en poids, d'un ou plusieurs cations métalliques.

29. Composition cosmétique, dermopharmaceutique ou pharmaceutique telle que définie à la revendication 28, dans laquelle le ou les cations métalliques sont choisis parmi les cations sodium, potassium, magnésium, manganèse ou aluminium.
